# EUROPEAN PATENT APPLICATION

(11) **EP 2 865 407 A1**
(43) Date of publication of application: **29.04.2015**
(21) Application number: 14174584.4
(22) Date of filing: 26.06.2014
(51) Int. Cl.: A61M 25/09, D07B 1/12, D07B 1/06

(54) **Coil body and guide wire**

(30) Priority: 25.10.2013 JP 2013222616
(71) Applicant: ASAHI INTECC CO., LTD., Nagoya-shi, Aichi 463-0024 (JP)
(72) Inventor: Sato, Kazuo c/o ASAHI INTECC CO., LTD., Nagoya-shi, Aichi 463-0024 (JP); Miyata, Naohiko c/o ASAHI INTECC CO., LTD., Nagoya-shi, Aichi 463-0024 (JP); Ikeno, Jun c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(57) **Abstract**

To provide a coil body that has high flexibility, satisfactorily tracks a complicated shape, and remarkably transmits fine vibrations occurring on a part of the coil body, and a guide wire using the coil body. A coil body 1 is formed by helically winding a stranded wire 11 including a plurality of stranded element wires. The stranded wire 11 has a first gap as a hollow at the center of the stranded wire.

## Description

### Technical Field

The present invention relates to a coil body having flexibility and a medical guide wire comprising the coil body.

### Background Art

Conventionally, a coil body having high flexibility has been used in various fields. For example, in the field of medical care, when a catheter is inserted into a blood vessel, an alimentary canal, a ureter or the like to conduct treatment and examinations, or when an indwelling device is inserted, a guide wire comprising a coil body on a distal portion of the guide wire is used to guide the catheter or the device.

Typically, the guide wire needs to be so flexible as to prevent damage to the interior walls of a blood vessel, an alimentary canal, and so on. A distal portion of the guide wire particularly needs high flexibility. For example, Patent Literature 1 discloses a guide wire that improves in flexibility with a shaft composed of stranded wires. Patent Literature 2 discloses a guide wire including a shaft composed of a core wire and a plurality of thin wires stranded around the core wire. Since the core wire is not provided on a distal portion of the shaft, the distal portion of the shaft has higher flexibility than a body portion of the shaft.

In recent years, the range of use of a guide wire tends to expand. Since blood vessels are particularly bent in a complicated manner, in many cases, a distal portion of a guide wire used for blood vessels needs to have higher flexibility and track blood vessels. As disclosed in Patent Literatures 1 and 2, a guide wire including a shaft composed of stranded wires has high flexibility. However, if thin wires constituting stranded wires are reduced in diameter to increase flexibility, stiffness of the guide wire and transmissibility of rotating torque of the guide wire decrease, leading to another problem: tracking of blood vessels may deteriorate or an operator who operates the a guide wire outside a body may not recognize detailed information, which includes a resistance when the distal portion of the guide wire is in contact with a wall surface or a stenosed part of a blood vessel in the body, from the guide wire, though the operator should recognize the information on the distal portion of the guide wire through a holding part.

### Citation List

### Patent Literatures

Patent Document 1: Japanese Patent Laid-Open No. 10-323395
Patent Document 2: Japanese Patent Laid-Open No. 2001-293092

### Summary of Invention

### Technical Problem

An object of the present invention is to provide a coil body that has high flexibility, satisfactorily tracks a complicated shape, and remarkably transmits fine vibrations occurring on a part of the coil body.

Another object of the present invention is to provide a guide wire that has high flexibility on the distal portion of the guide wire, satisfactorily tracks a complicated shape of a blood vessel and so on, and remarkably transmits detailed information on a resistance when the distal portion slides in contact with a wall surface of a blood vessel and so on or a resistance when the distal portion comes into contact with a stenosed part.

### Solution to Problem

A coil body according to the present invention is formed by helically winding a stranded wire including a plurality of stranded element wires, the stranded wire having a first gap as a hollow at the center of the stranded wire.

A guide wire according to the present invention includes a core shaft and the coil body covering a distal portion of the core shaft.

### Advantageous Effects of Invention

The coil body according to the present invention is formed by helically winding the stranded wire including the stranded element wires, the stranded wire has a hollow structure having the first gap instead of a core wire at the center of the stranded wire. With this configuration, the coil body of the present invention has high flexibility, smoothly moves so as to satisfactorily track a complicated shape, and remarkably transmits fine vibrations occurring on a part of the coil body. Furthermore, since the coil body has a smooth surface, even the coil body in contact with another member can smoothly slide.

The guide wire according to the present invention includes the core shaft and the coil body. The coil body is formed by helically winding the stranded wire including the stranded element wires. The stranded wire has a hollow structure having the first gap instead of a core wire at the center of the stranded wire. Since the guide wire comprises the coil body being formed by winding the stranded wire having the first gap at the center, the distal portion of the guide wire according to the present invention has high flexibility, smoothly moves so as to satisfactorily track a blood vessel and so on, and remarkably transmits detailed information on vibrations or the like when a resistance occurs on the distal portion. The coil body having a smooth surface allows the guide wire of the present invention to smoothly slide along a wall surface of a blood vessel, an alimentary canal, a ureter or the like.

### Brief Description of Drawings

FIG. 1 is a front view schematically showing an embodiment of the coil body according to the present invention.
FIG. 2 is a front view schematically showing an example of the stranded wire used in the embodiment of the present invention.
FIG. 3 is a cross-sectional view schematically showing an example of the stranded wire used in the embodiment of the present invention.
FIG. 4 is a cross-sectional view schematically showing another example of the stranded wire used in the embodiment of the present invention.
FIG. 5 is a cross-sectional view schematically showing another state of the stranded wire shown in FIG. 4.
FIG. 6 is a cross-sectional view schematically showing still another state of the stranded wire shown in FIG. 4.
FIG. 7 is a cross-sectional view schematically showing the embodiment of the coil body according to the present invention.
FIG. 8 is a cross-sectional view schematically showing another example of the embodiment of the coil body according to the present invention.
FIG. 9 is a cross-sectional view schematically showing an embodiment of the guide wire of the present invention.
FIG. 10 is a front view schematically showing a surface of a coil body of a comparative example.

### Description of Embodiments

An embodiment of the present invention will be described below with reference to the accompanying drawings. The present invention is not limited to the embodiment.

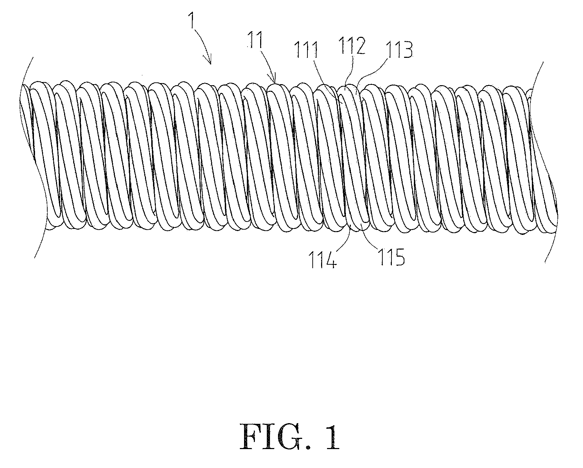

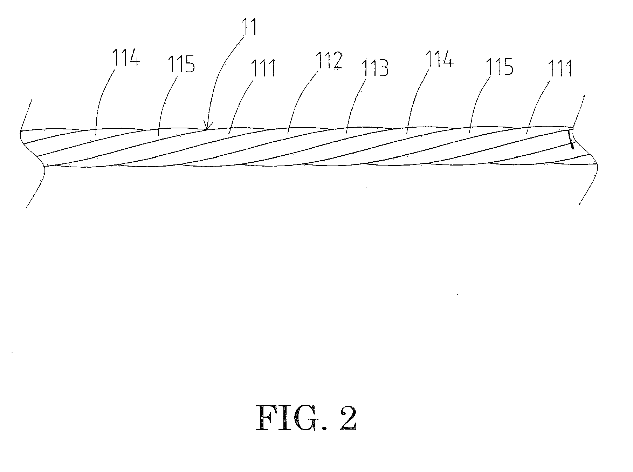

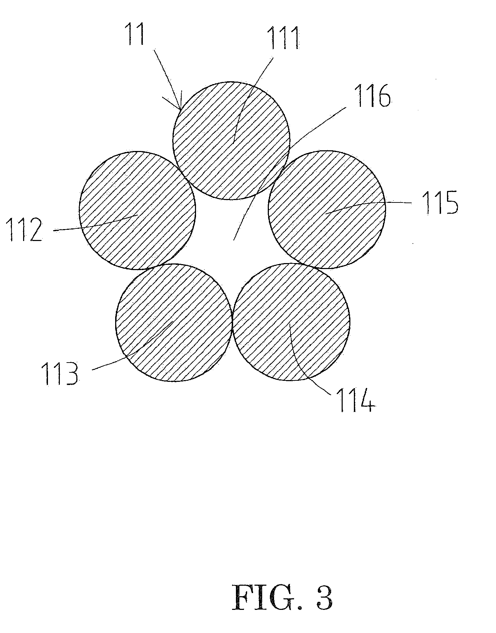

FIG. 1 is a front view schematically showing the embodiment of the coil body according to the present invention. FIG. 2 is a front view schematically showing a state of the stranded wire before the stranded wire are wound into the coil body. FIG. 3 is a cross-sectional view schematically showing a section of the stranded wire shown in FIG. 2.

A coil body 1 of the present invention is formed by helically winding a stranded wire 11 including a plurality of stranded element wires. The stranded wire 11 has a first gap at the center.

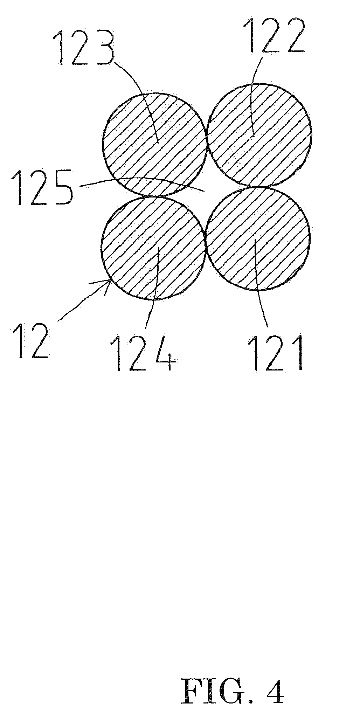

Preferably, a minimum number of the element wires constituting the stranded wire 11 is three, whereas a maximum number of the element wires is preferably eight and is more preferably six. In FIGS. 1, 2 and 3, the stranded wire 11 includes five element wires 111, 112, 113, 114 and 115 and a first gap 116 provided instead of a core wire at the center of the stranded wire 11. As shown in FIG. 4, the number of the element wires constituting the stranded wire is most preferably four. In FIG. 4, a stranded wire 12 includes four element wires 121, 122, 123 and 124 and a first gap 125 provided instead of a core wire at the center of the stranded wire 12.

In using the element wires within the above-mentioned range of the number, the first gap is reliably formed at the center of the obtained stranded wire and the stranded wire satisfactorily keeps the shape thereof. Further, when the stranded wire is wound in a helical fashion into the coil body, the coil body has a smooth surface.

The stranded wire is formed by stranding the element wires. The stranding method is not particularly limited as long as the obtained stranded wire has a hollow structure having the first gap at the center. Since the obtained stranded wire keeps a uniform shape with a smooth surface, the element wires are preferably stranded with regular pitches in the same direction in a helical fashion. In the case of the adjacent element wires, one of the element wires is preferably stranded over the other element wire in the direction in which the element wires are to be stranded (stranding direction).

Referring to FIG. 2, the stranded wire will be specifically described below. As shown in FIG. 2, the stranded wire 11 includes the five element wires 111, 112, 113, 114 and 115 that are stranded in a helical fashion in the same direction, that is, counterclockwise to the left in FIG. 2. More specifically, at a certain point, the five element wires 111, 112, 113, 114 and 115 are sequentially arranged like a circle counterclockwise to the left in FIG. 2. One of the element wires is stranded over the adjacent element wire counterclockwise (in the stranding direction). In short, at a certain point, the element wire 115 is located in the counterclockwise direction of the element wire 114, the element wire 114 is stranded over the element wire 115, the element wire 113 is stranded over the element wire 114, the element wire 112 is stranded over the element wire 113, the element wire 111 is stranded over the element wire 112 and the element wire 115 is stranded over the element wire 111. This process is repeated to obtain the hollow stranded wire 11 having the first gap 116 instead of a core wire at the center.

The five element wires were described in the above explanation. Four element wires may be stranded as shown in FIG. 4 in the same way or three or at least six element wires may be stranded in the same way. If the four element wires 121, 122, 123 and 124 are stranded, the hollow stranded wire 12 is obtained with the first gap 125 at the center.

The element wire is made of materials including stainless steel and superelastic alloys such as a Ni-Ti alloy. The element wires may be made of the same material or the element wires made of different materials may be combined.

The element wires may have circular, oval, or polygonal shapes such as a square and a rectangle in cross section. In consideration of ease of stranding, the maintenance of the shape of the obtained stranded wire, and the smoothness of the surface of the obtained stranded wire, the element wires are preferably circular in cross section.

If the element wires are circular in cross section, as shown in FIGS. 3 and 4, the element wires preferably have a nearly equal diameter. In this case, the element wires preferably have a maximum diameter of 0.05 mm and a minimum diameter of 0.01 mm.

The stranded wires 11 and 12 have the first gaps 116 and 125 instead of core wires at the centers. The relative positional relationship among the element wires constituting the stranded wire is changeable according to a force applied to the stranded wire. For example, when the stranded wires 11 and 12 are wound in a helical fashion, the linear or curved stranded wires 11 and 12 are deformed into helical shapes. The relative positional relationship among the element wires can be slightly changed according to the deformation. Thus, the stranded wires 11 and 12 can be easily wound into helical shapes and the coil body obtained by winding the element wires in a helical fashion is less likely to have an uneven surface. If the coil body obtained by winding the stranded wires 11 and 12 into helical shapes is deformed or if the coil body used for a guide wire or the like that is moved in curved blood vessels, the coil body is slightly deformed. Also in this case, the relative positional relationship among the element wires constituting the stranded wires 11 and 12 is slightly changed to suppress a force against the deformation of the coil body. Hence, the coil body has high flexibility and satisfactorily tracks a complicated shape.

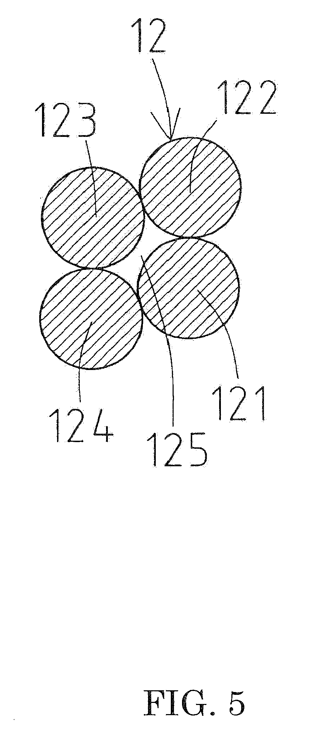

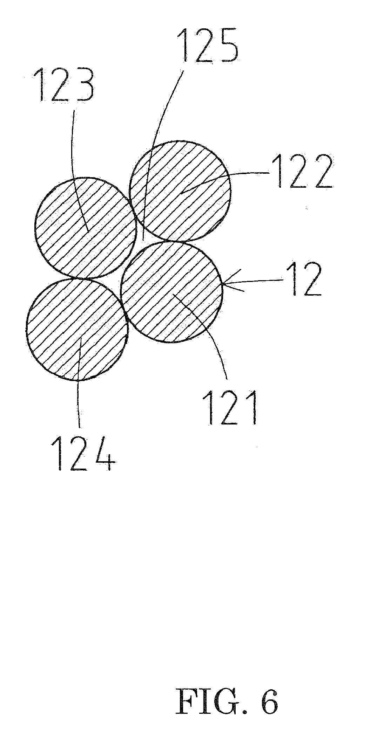

FIGS. 4, 5 and 6 are cross-sectional views for schematically illustrating the relative positional relationship among the element wires of the stranded wire 12. The stranded wire 12 includes the four element wires 121, 122, 123 and 124. The stranded wire 12 in the state of FIG. 4 is slightly deformed so as to slightly change the relative positional relationship among the element wires 121, 122, 123 and 124 according to the deformation of the stranded wire 12 as shown in FIG. 5. When the stranded wire 12 is further deformed, the relative positional relationship among the element wires 121, 122, 123 and 124 is further changed as shown in FIG. 6. The relative positional relationship among the element wires is slightly changed according to the deformation of the stranded wire, reducing a force against the deformation.

Furthermore, since the element wires are stranded each other, even if the relative positional relationship is slightly changed, the stranded element wires are not separated from one another, preventing deformation of the stranded wire and the coil body including the stranded wire wound in a helical fashion.

As described above, the stranded wire having the first gap at the center is highly flexible in response to deformation, allowing the coil body including the stranded wire wound in a helical fashion to have unique excellent effects, e.g., a smooth surface, high slidability, high flexibility, and excellent tracking of complicated shapes.

As shown in FIG. 1, the stranded wire 11 is wound in a helical fashion to form the coil body 1.

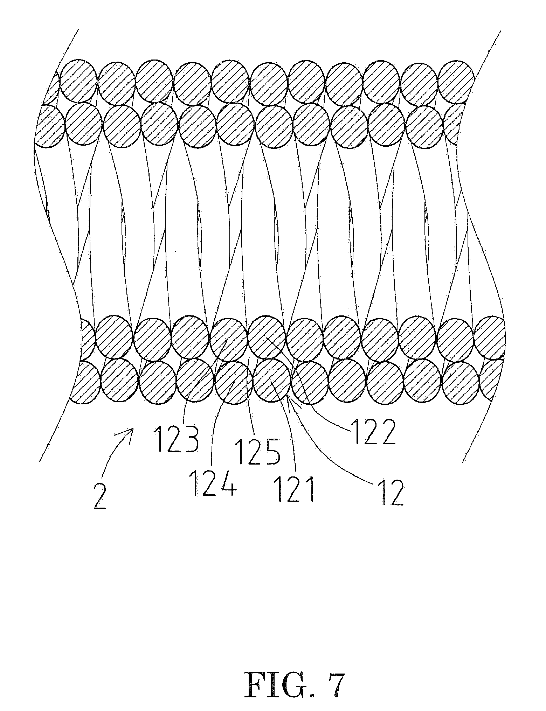

FIG. 7 is a cross-sectional view schematically showing a longitudinal section of the coil body 2 formed by winding the stranded wire 12 in a helical fashion, the stranded wire 12 including the four element wires shown in FIG. 4.

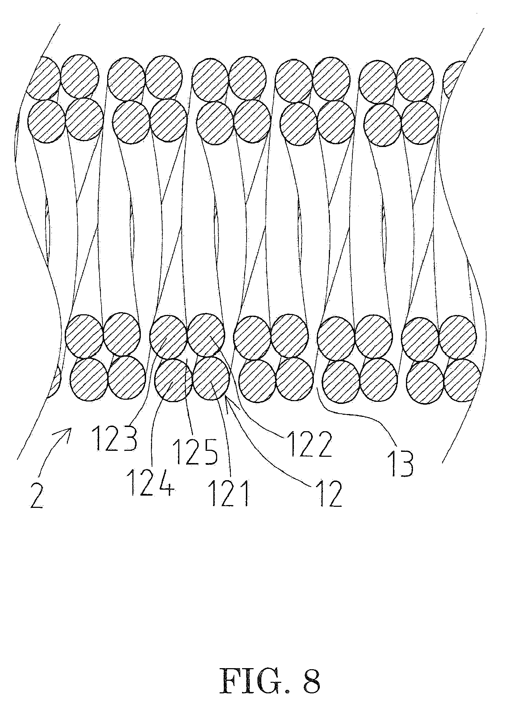

As shown in FIG. 7, the stranded wire 12 wound in intimate contact with the adjacent stranded wire. FIG. 8 shows another preferable configuration in which the stranded wires wound in a helical fashion are not in close contact with each other with a second gap 13 provided between the adjacent stranded wires. The stranded wires constituting the coil body 2 are not in close contact with each other with the second gap 13 provided between the adjacent stranded wires, further improving the flexibility of the coil body 2, tracking of complicated shapes, and transmission of fine vibrations occurring on a part of the coil body.

The relationship between the stranding direction of the stranded wire and the winding direction of the coil body is not particularly limited. For example, if the stranded wire including the element wires stranded counterclockwise is wound in a helical fashion to form the coil body, the coil body may be helically formed clockwise or counterclockwise. If the stranded wire including the element wires stranded clockwise is wound in a helical fashion to form the coil body, the coil body may be helically formed clockwise or counterclockwise.

The coil body 2 may be fully composed of the stranded wire 12 or partially composed of the stranded wire 12. If the coil body 2 is partially composed of the stranded wire 12, the configuration of the coil body 2 may be optionally adjusted depending upon the purpose and usage method of the coil body. For example, only a necessary part of the coil body is composed of the stranded wire 12. In the case of the coil body attached to a distal portion of a medical guide wire, for example, the coil body typically has a maximum length of 500 mm, preferably 400 mm, and typically has a minimum length of 100 mm, preferably 200 mm. In this case, if the distal side of the guide wire is located at the distal side of the coil body, the stranded wire 12 is preferably forming at least in the portion including the distal end of the coil body, preferably at least 20 mm from the distal end of the coil body, more preferably at least 30 mm from the distal end of the coil body. If the coil body 2 is partially composed of the stranded wire 12, the other part is optionally composed of a known coil body.

The coil bodies 1 and 2 are used for various purposes requiring flexibility and tracking of complicated shapes. The coil bodies 1 and 2 are particularly satisfactorily used as coil bodies attached to the distal portions of medical guide wires.

The present invention includes a guide wire provided with the coil body according to the present invention attached over the distal portion of a core shaft.

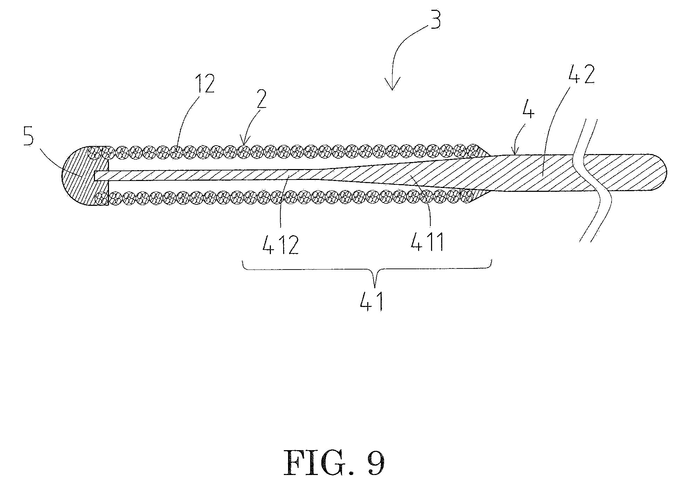

FIG. 9 is a cross-sectional view schematically showing an embodiment of the guide wire according to the present invention. The left side of FIG. 9 is located on the distal end to be inserted into a body (distal side) while the right side is located on a proximal end (proximal side) operated by an operator.

A guide wire 3 is used for cardiovascular therapy and so on includes a core shaft 4 with an distal portion 41 covered with the coil body 2.

The guide wire 3 is a long wire having flexibility. The length and diameter of the guide wire 3 are optionally set depending upon the usage range of the guide wire 3. The guide wire 3 typically has a maximum length of 4000 mm, preferably 2500 mm, and typically has a minimum length of 500 mm, preferably 1000 mm. Moreover, the guide wire 3 typically has a maximum diameter of at least 0.05 mm to 0.5 mm in cross section.

The core shaft 4 is a long shaft having flexibility. The length and the maximum diameter of the core shaft 4 are set according to the length and the maximum diameter of the guide wire 3. The core shaft 4 may have circular, oval, or polygonal shapes such as a square and a rectangle in cross section. The core shaft 4 is typically and preferably circular in cross section.

The core shaft 4 includes an distal portion 41 located on the distal side of the core shaft 4 so as to be inserted into a body during treatment, an examination, and so on, and a main portion 42 located on the proximal side of the distal portion 41.

On the distal portion 41, the core shaft 4 gradually decreases in diameter and increases in flexibility toward the distal end. For example, if the guide wire 3 is used for cardiovascular therapy, the distal portion 41 is typically provided from the distal end to 400 mm toward the proximal side of the guide wire 3 in the axial direction.

In FIG. 9, the distal portion 41 includes a tapered portion 411 and a small-diameter portion 412. The distal portion 41 includes the tapered portion 411 decreasing in diameter toward the distal end and thus also gradually decreases in diameter toward the distal end. The small-diameter portion 412 extends with a substantially constant diameter from the distal end of the tapered portion 411 in the axial direction. The distal end of the small-diameter portion 412 is joined to the distal end of the coil body 2 via joining means. The joining means is not particularly limited as long as the joining means has a smooth shape that prevents the distal end of the guide wire 3 from damaging a wall surface of a blood vessel. A known joining method may be used. Typically, as shown in FIG. 9, the distal end of the small-diameter portion 412 is joined via a distal-end chip 5 having a smooth distal end face.

The distal-end chip 5 has a smooth distal end face having a hemispheric face that does not damage blood vessels and so on. The forming method of the distal-end chip 5 is not particularly limited as long as the distal end of the core shaft 4 and the distal end of the coil body 2 are joined to each other. For example, preferably, members such as the core shaft 4 and the coil body 2 are combined, and then the distal ends thereof are coated with a brazing filler metal to join the distal ends of the core shaft 4 and the coil body 2 by brazing, forming the distal-end chip 5 by means of the used brazing filler metal.

In the guide wire 3 of FIG. 9, the distal portion 41 includes the single tapered portion 411 and the single small-diameter portion 412. The configuration of the guide wire 3 is not particularly limited. The tapered portion may be provided over the distal portion or a plurality of tapered portions may be provided. For example, the following configuration is applicable: a first tapered portion decreasing in diameter toward the distal end, a first small-diameter portion extending with substantially a constant diameter in the axial direction, a second tapered portion decreasing in diameter toward the distal end, and a second small-diameter portion that extends with substantially a constant diameter in the axial direction and is joined to the distal-end chip are sequentially provided from the proximal side of the distal portion.

In the guide wire 3 of FIG. 9, the distal end of the distal portion 41 is joined to the distal-end chip 5 while the distal end of the distal portion 41 is separated from the distal-end chip 5 without joining the distal portion 41 and the distal-end chip 5. In this case, the core shaft and the distal-end chip are preferably connected via a safety wire or the like. Also in the case where the distal portion 41 and the distal-end chip 5 are joined to each other, the core shaft and the distal-end chip may be further connected via a safety wire or the like.

The main portion 42 of the core shaft 4 extends with substantially a constant diameter in the axial direction from the proximal end of the distal portion 41. The main portion 42 is a part other than the distal portion 41 of the core shaft 4. The distal side of the main portion 42 is inserted into a body following the distal portion 41 during treatment or an examination, whereas the proximal side of the main portion 42 remains exposed out of the body.

The core shaft 4 is made of known materials including stainless steel, superelastic alloys such as a Ni-Ti alloy, and piano wires. Stainless steel is particularly preferable. The overall core shaft 4 may be made of the same material or a part of the core shaft 4 may be made of a different material.

The distal end of the coil body 2 is joined to the distal-end chip 5 while the proximal end of the coil body 2 is joined to the core shaft 4 around the proximal end of the distal portion 41 of the core shaft 4 by known joining methods such as brazing, soldering, and bonding with an adhesive.

The manufacturing method of the guide wire is not particularly limited. For example, the guide wire is manufactured as follows:

First, the element wires are stranded to form the stranded wire, the obtained stranded wire is helically wound around a core having a desired diameter, and then the core is removed to fabricate the coil body. After that, the core shaft molded into a desired shape is inserted into the coil body. The coil body and the core shaft are aligned with each other, and then the proximal end of the coil body and the core shaft are joined by known joining methods such as brazing, soldering, and bonding with an adhesive. Subsequently, the distal ends of the coil body and the core shaft are joined with the brazing filler metal or the like. When the distal ends of the coil body and the core shaft are joined with the brazing filler metal, the distal-end chip is formed.

In the following example, the method of using the guide wire 3 for treatment or an examination according to the embodiment of the present invention is used for a stenosed part formed in a coronary artery.

The guide wire 3 is inserted into an artery from a femoral region, passes through an aortic arch, and then moves to a stenosed part formed in a coronary artery to be treated. In this process, a pressing force or a rotary force is applied to the guide wire 3 from an operator, e.g., a doctor. The guide wire 3 has excellent flexibility on the distal portion and satisfactorily tracks a blood vessel. This allows the guide wire 3 to smoothly track a blood vessel curved in a complicated manner, without damaging a blood vessel wall. Thus, the guide wire 3 is smoothly moved to the stenosed part. Moreover, the guide wire 3 satisfactorily transmits detailed information on a resistance when the distal portion slides in contact with a wall surface of a blood vessel and so on or a resistance when the distal portion comes into contact with a stenosed part. Thus, an operator will move the guide wire while recognizing a resistance applied to the distal portion.

After the guide wire 3 reaches a part to be treated, a treatment catheter such as a balloon catheter and a catheter for introducing a treatment member is inserted into a body along the guide wire 3 to conduct treatment, e.g., dilation of a stenosed part.

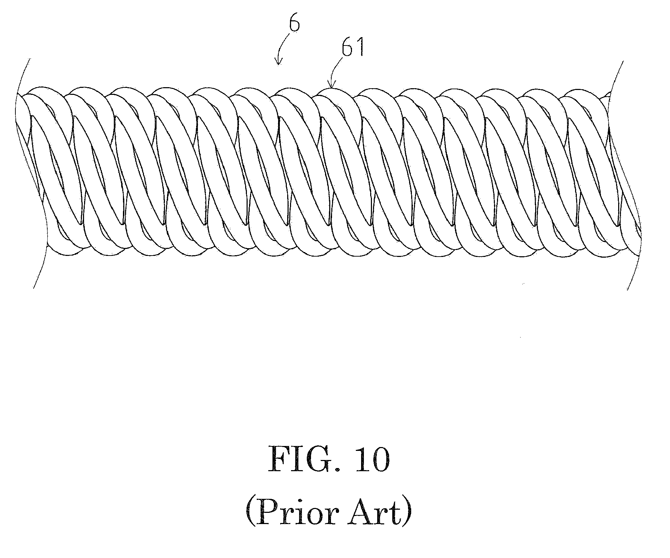

For comparison, FIG. 10 is a front view schematically showing the surface of a coil body including a known stranded wire having a core wire instead of a gap at the center.

A coil body 6 is formed by winding a known stranded wire 61 in a helical fashion.

The known stranded wire 61 includes five element wires that are composed of stainless steel and are circular with a diameter of about 0.03 mm in cross section and a core wire (element wire) that is composed of stainless steel and is circular with a diameter of about 0.015 mm in cross section. The five element wires are helically stranded around the core wire. The stranded wire is stranded by the same manner as that of the core body 1 according to the embodiment of the present invention, except for the use of the core wire.

FIG. 10 shows the surface of the coil body 6 composed of the stranded wire 61 having a core wire at the center. In the known stranded wire 61, the relative positional relationship among the element wires is less likely to be changed as compared with the stranded wires 11 and 12 used in the present invention. Thus, a deformation which arises among the element wires when the element wires are stranded into the stranded wire 61 and a deformation which arises among the element wires when the stranded wire 61 is wound in a helical fashion are increased compared to the stranded wires 11 and 12 of the invention. This increases the ratio of the element wires protruding from the surface of the coil body 6, causing irregularities on the surface of the coil body 6 as compared with the surface of the coil body 1 of FIG. 1 according to the present invention.

### Reference Signs List

- 1, 2, 6: coil body
- 11, 12, 61: stranded wire
- 111, 112, 113, 114, 115, 121, 122, 123, 124: element wire
- 116, 125: first gap
- 13: second gap
- 3: guide wire
- 4: core shaft
- 41: distal portion
- 411: tapered portion
- 412: small-diameter portion
- 42: main portion
- 5: distal-end chip

## Claims

1. A coil body (1, 2) formed by helically winding a stranded wire (11, 12) including a plurality of stranded element wires (111, 112, 113, 114, 115, 121, 122, 123, 124),
the stranded wire (11, 12) having a first gap (116, 125) as a hollow at a center of the stranded wire.

2. The coil body (2) according to claim 1, wherein a second gap (13) is formed between adjacent portions of the stranded wire (12) wound in a helical fashion.

3. The coil body (2) according to claim 1 or 2, wherein the number of the element wires forming the stranded wire (12) is four.
